(19) Europäisches Patentamt
European Patent Office
Office européen des brevets

(11) Publication number: 0 108 526 A2

# (12) EUROPEAN PATENT APPLICATION

(21) Application number: 83306301.9

(22) Date of filing: 18.10.83

(51) Int. Cl.³: C 12 Q 1/28

(30) Priority: 01.11.82 US 438198

(43) Date of publication of application: 16.05.84 Bulletin 84/20

(84) Designated Contracting States: DE FR GB IT

(71) Applicant: BECKMAN INSTRUMENTS, INC.
2500 Harbor Boulevard
Fullerton California 92634(US)

(72) Inventor: Chen, Albert Fu-Tai
1451 Bradshawe Avenue
Monterey Park California 91754(US)

(74) Representative: Huskisson, Frank Mackie et al,
FITZPATRICKS 4 West Regent Street
Glasgow G2 1RS Scotland(GB)

## (54) Method and reagent for the determination of peroxide and precursors thereof.

(57) An improved colorimetric reagent for the determination of peroxide in a sample. The reagent is of the type comprising a peroxide activating agent, a hydrogen donor, and an electron or radical acceptor. The reagent is characterized in that the hydrogen donor is a compound having a formula

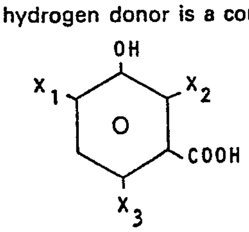

wherein $X_1$, $X_2$, and $X_3$, when taken separately, are selected from a group consisting of hydrogen and halogen.

Also, an improved method for determining peroxide in a sample. The method is of the type wherein a hydrogen donor is oxidatively condensed with an electron or radical acceptor in the presence of a peroxide activating agent to produce a color developed material whose optical absorbance is capable of being determined. The improvement comprising employing the above described compound as the hydrogen donor therein.

EP 0 108 526 A2

# METHOD AND REAGENT FOR THE DETERMINATION OF PEROXIDE AND PRECURSORS THEREOF

## Background of the Invention

### 1. Field of the Invention

This invention relates to an improved method for the determination of peroxide and a reagent for use therein.

### 2. Description of the Prior Art

In a widely used method, the quantity of a given compound, e.g., cholesterol, in a sample is determined by treating a sample with an oxidase and measuring the amount of hydrogen peroxide formed. Generally, hydrogen peroxide is quantitatively determined by a method wherein the hydrogen peroxide is reacted with a colorimetric reagent comprising a peroxidase, a hydrogen donor, and an electron or radical acceptor which react together stoichiometrically with the hydrogen peroxide to form a pigment. The amount of hydrogen peroxide is then calculated from the relationship between the amount of pigment formed and the absorbancy at a visible portion of the spectrum.

As the hydrogen donor, the colorimetric reagent generally contains a compound such as phenol, dimethylaniline, diethylaniline, o-tolidine, o-toluidine, p-toluidine, o-phenylenediamine, N,N'-dimethyl-p-phenylenediamine, benzidine, o-anisidine, p-anisidine, dianisidine, o-cresol, m-cresol, α-naphthol, β-naphthol, catechol, guaiacol, pyrogallol, 2,7-diaminofluorene, leucoindophenol, 2,4-dichlorophenol, or 2-hydroxyl-3,5-dichlorobenzenesulfonic acid.

As the electron or radical acceptor, the colorimetric reagent generally contains a compound such as 2-thiophenecarboxylic acid hydrazine, benzidine, 3-methyl-2-benz-thiazolinone hydrazone, and a 4-aminoantipyrine represented by the formula

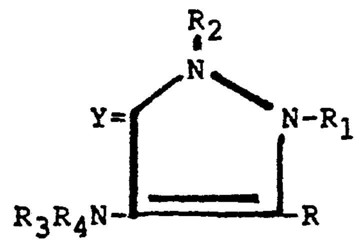

wherein each of R and $R_1$, when taken separately, is alkyl, $R_2$ is an aromatic moiety, each of $R_3$ and $R_4$, when taken separately, is selected from a group consisting of hydrogen and alkyl, and Y is selected from a group consisting of oxygen and sulfur.

As the peroxide activiting agent, the colorimetric reagent generally contains a compound selected from a group consisting of peroxidase, tungstic acid, salts of tungstic acid, molybdic acid, salts of molybdic acid, a mixture of an iodide and one of these inorganic compounds, and ions of iron, copper, cerium, and vanadic acid.

Of the hyrogen donors listed above, only phenol, 3,4-dichlorophenol, and 2-hydroxyl-3,5-dichlorobenzenesulfonic acid are commonly employed in commercial colorimetric reagents. These hydrogen donors have various disadvantages, e.g., unpleasant odor (phenol and 2,4-dichlorophenol), poor stability, (2-hydroxyl-3,5-dichlorobenzenesulfonic acid), or relatively low sensitivity (phenol).

Although a considerable amount of research has been carried out in this area (1-5), there is still a

need for an improved hydrogen donor for use in a colorimetric reagent for the determination of peroxide or a precursor thereof.

## Bibliography


1. Ueno et al., J. Clin. Chem. Clin. Biochem., 19 8:861 (1981)
2. Chemical Abstracts, 95:93423q (1981)
3. U.S. Patent 4,247,631
4. U.S. Patent 4,251,629
5. U.S. Patent 4,260,679


## Summary of the Invention

In accordance with the present invention, there is provided an improved colorimetric reagent for the determination of peroxide in a sample. The improved colorimetric reagent of the instant invention is of the type comprising a peroxide activating agent, a hydrogen donor, and an electron or radical acceptor. The improved colorimetric reagent of the instant invention is characterized in that the hydrogen donor is a compound having a formula

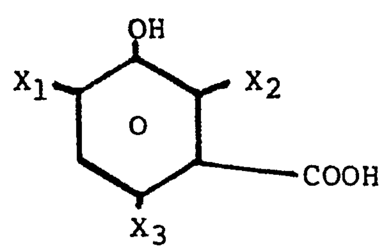

wherein $X_1$, $X_2$, and $X_3$ are, when taken separately, selected from a group consisting of hydrogen and halogen.

In addition, the present invention also encompasses an improved method for determining peroxide in a sample. The improved method of the instant invention is of the type wherein a hydrogen donor is oxidatively

condensed with an electron or radical acceptor in the presence of an oxide activating agent to produce a color developed material whose optical absorbance is capable of being determined. The method of the instant invention is characterized in that the method employs the hydrogen donor described above.

## Description of the Preferred Embodiments

The compounds illustrated in Table I may be used as the hydrogen donor of the instant invention.

Table I

| Compound Number | $X_1$ | $X_2$ | $X_3$ |
|---|---|---|---|
| 1 | H | H | H |
| 2 | Cl | H | H |
| 3 | Br | H | H |
| 4 | I | H | H |
| 5 | F | H | H |
| 6 | H | Cl | H |
| 7 | H | Br | H |
| 8 | H | I | H |
| 9 | H | F | H |
| 10 | H | H | Cl |
| 11 | H | H | Br |
| 12 | H | H | F |
| 13 | H | H | I |
| 14 | Cl | Cl | H |
| 15 | Cl | Br | H |
| 16 | Br | Cl | H |
| 17 | Cl | I | H |
| 18 | I | Cl | H |
| 19 | Cl | F | H |
| 20 | F | Cl | H |
| 21 | Br | Br | H |

| | | | |
|---|---|---|---|
| 22 | Br | I | H |
| 23 | I | Br | H |
| 24 | Br | F | H |
| 25 | F | Br | H |
| 26 | I | I | H |
| 27 | I | F | H |
| 28 | F | I | H |
| 29 | F | F | H |
| 30 | Cl | H | Cl |
| 31 | Cl | H | Br |
| 32 | Br | H | Cl |
| 33 | Cl | H | I |
| 34 | I | H | Cl |
| 35 | Cl | H | F |
| 36 | F | H | Cl |
| 37 | Br | H | Br |
| 38 | Br | H | I |
| 39 | I | H | Br |
| 40 | Br | H | F |
| 41 | F | H | Br |
| 42 | I | H | I |
| 43 | I | H | F |
| 44 | F | H | I |
| 45 | F | H | F |
| 46 | H | Cl | Cl |
| 47 | H | Cl | Br |
| 48 | H | Br | Cl |
| 49 | H | Cl | I |
| 50 | H | I | Cl |
| 51 | H | Cl | F |
| 52 | H | F | Cl |
| 53 | H | Br | Br |
| 54 | H | Br | I |
| 55 | H | I | Br |
| 56 | H | Br | F |
| 57 | H | F | Br |

| | | | |
|---|---|---|---|
| 58 | H | I | I |
| 59 | H | I | F |
| 60 | H | F | I |
| 61 | H | F | I |
| 62 | Cl | Cl | Cl |
| 63 | Cl | Cl | Br |
| 64 | Cl | Cl | I |
| 65 | Cl | Cl | F |
| 66 | Cl | Br | Cl |
| 67 | Cl | I | Cl |
| 68 | Cl | F | Cl |
| 69 | Br | Cl | Cl |
| 70 | I | Cl | Cl |
| 71 | F | Cl | Cl |
| 72 | Br | Br | Br |
| 73 | Br | Br | Cl |
| 74 | Br | Br | I |
| 75 | Br | Br | F |
| 76 | Br | Cl | Br |
| 77 | Br | I | Br |
| 78 | Br | F | Br |
| 79 | Cl | Br | Br |
| 80 | I | Br | Br |
| 81 | F | Br | Br |
| 82 | I | I | I |
| 83 | I | I | Cl |
| 84 | I | I | Br |
| 85 | I | I | F |
| 86 | I | Cl | I |
| 87 | I | Br | I |
| 88 | I | F | I |
| 89 | Cl | I | I |
| 90 | Br | I | I |
| 91 | F | I | I |
| 92 | F | F | F |
| 93 | F | F | Cl |

| | | | |
|---|---|---|---|
| 94 | F | F | Br |
| 95 | F | F | I |
| 96 | F | Cl | F |
| 97 | F | Br | F |
| 98 | F | I | F |
| 99 | Cl | F | F |
| 100 | Br | F | F |
| 101 | I | F | F |
| 102 | Cl | Br | I |
| 103 | Cl | I | Br |
| 104 | Cl | Br | F |
| 105 | Cl | F | Br |
| 106 | Cl | I | F |
| 107 | Cl | F | I |
| 108 | Br | Cl | I |
| 109 | I | Cl | Br |
| 110 | Br | Cl | F |
| 111 | F | Cl | Br |
| 112 | I | Cl | F |
| 113 | F | Cl | I |
| 114 | Br | I | Cl |
| 115 | I | Br | Cl |
| 116 | Br | F | Cl |
| 117 | F | Br | Cl |
| 118 | I | F | Cl |
| 119 | F | I | Cl |
| 120 | Br | I | F |
| 121 | Br | F | I |
| 122 | I | Br | F |
| 123 | F | Br | I |
| 124 | I | F | Br |
| 125 | F | I | Br |

The colorimetric reagent and method of the instant invention can be used in the determination of a peroxide having a formula $ZOOZ_1$, wherein each of Z and

$Z_1$, when taken separately, is selected from a group consisting of hydrogen and alkyl. More preferably, the colorimetric reagent and method of the instant invention are used for the determination of hydrogen peroxide.

In a particular aspect of the present invention, the above-described colorimetric reagent and method are used in the quantitative determination of precursor materials such as glucose, uric acid, cholesterol, choline esterase, phospholipid, or creatinine. These precursor materials can be converted incrematically and quantitatively into hydrogen peroxide using an oxidase enzyme. To this end, there may be included in the colorimetric reagent of this invention an oxidase capable of quantitatively converting a precursor material into hydrogen peroxide, whereby the amount of such precursor material present in the original sample may be determined. Correspondingly, the present invention also includes a method of determining the amount of such precursor material in a sample by treating that precursor material with an oxidase and quantitatively determining the amount of hydrogen peroxide produced by a method as above defined using the compounds for Formula I as the hydrogen donor.

The method and reagent of this invention may thus be used in the determination of cholesterol, phospholipid, glucose, uric acid, etc., by initial treatment of the sample with an enzyme such as, respectively, a cholesterol oxidase, a choline oxidase, a glucose oxidase, or a uricase followed by colorimetric determination of the hydrogen peroxide used by measuring the visual absorbancy of the sample when subsequently treated with the colorimetric reagent, i.e., the peroxidase in combination with the hydrogen donor and the electron or radical acceptor.

In the present invention, the hydrogen donor is used in an amount enough to react with hydrogen peroxide present or formed in situ in the sample. This amount is generally 2 to 5 equivalents to hydrogen peroxide. The absorbancy of the colored solution produced is then measured at a visible portion of from about 490 to about 530 nm and the amount of hydrogen peroxide is calculated from a standard curve for a standard sample.

The present invention also provides a kit consisting of an oxidase capable of reacting with a precursor material to produce hydrogen peroxide, and a colorimetric pick-up system for quantitatively converting the formed hydrogen peroxide into a colorimetrically detectable product.

The following examples are provided for the purpose of further illustration only and are not intended to be limitations on the disclosed invention.

Example 1

Reagent Within the Scope of Invention

A reagent containing the following ingredients was prepared:

| Ingredients | Concentration |
|---|---|
| cholesterol esterase | 1 IU/ml |
| cholesterol oxidase | 0.5 IU/ml |
| peroxidase (horseradish) | 100 IU/ml |
| Na-cholate | 6.9 mM |
| 4-aminoantipyrine | 1.6 mM |
| m-hydroxy benzoic acid | 5 mM |
| buffer (sodium phosphate) | 100 mM |
| pH | variable |

## Example 2

## Reagent Outside the Scope of Invention

A reagent containing the same ingredients with one modification was prepared. The sole modification was the use of 5 mM phenol in place of the 5 mM m-hydroxy benzoic acid.

Phenol is hydroscopic and possesses an unpleasant, strong, pungent odor. In contrast, m-hydroxy benzoic acid and the other hydrogen donors listed in Table I do not possess any unpleasant odor and are not hydroscopic. Accordingly, the hydrogen donors suggested for use in the present invention are easier to work with due to their different physical properties.

## Example 3

## Assay Procedure Within Scope of Invention

An aliquot (1 ml) of the reagent prepared in Example 1 was placed in a spectrophotometric cuvette. The optical density (OD) thereof was read at 500 nm. An aliquot (10 µl) of a calibrator (200 mg/dl cholesterol) was then introduced into the cuvette and mixed. The reaction was incubated at 30° C. and allowed to each an end-point. After about 10 minutes the OD was read. The data obtained from this experiment is set forth in Table II.

## Example 4

## Assay Procedure Outside Scope of Invention

The methodology employed in Example 3 was repeated using the reagent of Example 2. The data obtained is also set forth in Table II.

Table II

| pH | ΔA |
|---|---|
| Reagent Within Scope of Invention (Example 3) | |
| 6.5 | 0.320 |
| 7.0 | 0.385 |
| 7.5 | 0.395 |
| Reagent Outside Scope of Invention (Example 4) | |
| 7.5 | 0.330 |

The data set forth in Table II indicate that one is able to obtain an increase in sensitivity of approximately 20% by using a reagent system differing solely in that, instead of employing phenol, the hydrogen donor employed therein is one within the scope of the instant invention, namely, m-hydroxy benzoic acid.

Based on this disclosure, many other modifications and ramifications will naturally suggest themselves to those skilled in the art. These are intended to be comprehended as within the scope of this invention.

CLAIMS

1. An improved colorimetric reagent for the determination of peroxide in a sample of the type comprising a peroxide activating agent, a hydrogen donor, and an electron or radical acceptor, characterized in that said hydrogen donor is a compound having a formula I

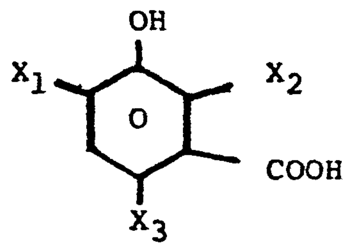

I

wherein $X_1$, $X_2$, and $X_3$, when taken separately, are selected from a group consisting of hydrogen and halogen.

2. The improved reagent of claim 1 wherein $X_1$, $X_2$, and $X_3$, when taken separately, are selected from a group consisting of hydrogen and chlorine, provided that at least one of $X_1$, $X_2$, or $X_3$ is chlorine.

3. The improved reagent of claim 1 wherein $X_1$ is halogen and $X_2$ and $X_3$ are hydrogen.

4. The improved reagent of claim 1 wherein $X_3$ is halogen and $X_1$ and $X_2$ are hydrogen.

5. The improved reagent of claim 1 wherein $X_1$ is chlorine and $X_2$ and $X_3$ are hydrogen.

6. The improved reagent of claim 1 wherein $X_3$ is chlorine and $X_1$ and $X_3$ are hydrogen.

7. The improved reagent of any one of claims 1, 2, 3, 4, 5, or 6 wherein

(a) said electron or radical acceptor is selected from a group consisting of 2-thiophenecarboxylic acid hydrazide, benzidine, 3-methyl-2-benzthiazolinone hydrazone, and a 4-aminoantipyrine represented by the formula

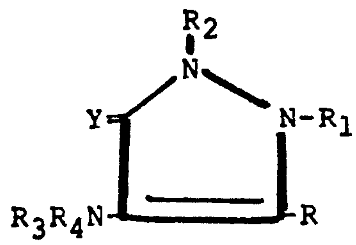

wherein each of R and $R_1$, when taken separately, is alkyl, $R_2$ is an aromatic moiety, each of $R_3$ and $R_4$, when taken separately, is selected from a group consisting of hydrogen and alkyl, and Y is selected from a group consisting of oxygen and sulfur;

(b) said peroxide has a formula $ZOOZ_1$ wherein each of Z and $Z_1$, when taken separately, is selected from a group consisting of hydrogen and alkyl; and

(c) said peroxide activating agent is selected from a group consisting of peroxidase, tungstic acid, salts of tungstic acid, molybdic acid, salts of molybdic acid, a mixture of an iodide and one of these organic compounds, and ions of iron, copper, cerium, and vanadic acid.

8. The improved reagent of any one of claims 1, 2, 3, 4, 5, or 6 wherein

(a) said electron or radical acceptor is 4-aminoantipyrine;

(b) said peroxide is hydrogen peroxide; and

(c) said peroxide activating agent is peroxidase.

9. The improved reagent of any one of claim 1, 2, 3, 4, 5, or 6 further comprising an oxidase capable of quantitatively forming peroxide in situ in the sample from a precursor material, whereby said precursor material may be quantitatively determined.

10. The improved reagent of any one of claims 1, 2, 3, 4, 5, or 6 further comprising an oxidase selected from a group consisting of cholesterol oxidase, choline oxidase, glucose oxidase, and uricase, said oxidase being capable of quantitatively forming peroxide in situ in a sample from a precursor material selected from a group consisting of cholesterol, phospholipid, glucose, and uric acid, whereby said precursor material may be quantitatively determined.

11. The improved reagent of any one of claims 1, 2, 3, 4, 5, or 6

(a) further comprising an oxidase capable of quantitatively forming peroxide in situ in the sample from a precursor material, whereby said precursor material may be quantitatively determined;

(b) said electron or radical acceptor is selected from a group consisting of 2-thiophenecarboxylic acid hydrazide, benzidine, 3-methyl-2-benzthiazolinone hydrazone, and a 4-aminoantipyrine represented by the formula

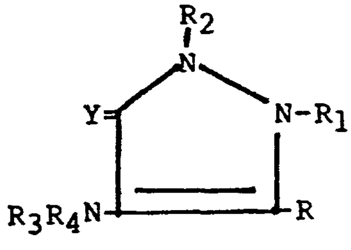

wherein each of R and $R_1$, when taken separately, is alkyl, $R_2$ is an aromatic moiety, each of $R_3$ and $R_4$, when taken separately, is selected from a group consisting of hydrogen and alkyl, and Y is selected from a group consisting of oxygen and sulfur;

(c) said peroxide has a formula $ZOOZ_1$ wherein each of Z and $Z_1$, when taken separately, is slected from a group consisting of hydrogen and alkyl; and

(d) said peroxide activating agent is selected from a group consisting of peroxidase, tungstic acid, salts of tungstic acid, molybdic acid, salts of molybdic acid, a mixture of an iodide and one of these organic compounds, and ions of iron, copper, cerium, and vanadic acid.

12. The improved reagent of any one of claims 1,2,3,4, 5, or 6

(a) further comprising an oxidase selected from a group consisting of cholesterol oxidase, choline oxidase, glucose oxidase, and uricase, said oxidase being capable of quantitatively forming peroxide in situ in a sample from a precursor material selected from a group consisting of cholesterol, phospholipid, glucose, and uric acid, whereby said precursor material may be quantitatively determined; and wherein

(b) said electron or radical acceptor is 4-aminoantipyrine;

peroxidase.

13. A method for determining peroxide in a sample comprising reacting said sample with a reagent as claimed in any one of claims 1 to 12 thereby to produce a color developed material and determining

the optical absorbance of the color characterised in that the said hydrogen donor is a compound having a formula I defined on any of claims 1 to 6.

14. A method as claimed in claim 13, wherein said electron or radical acceptor is as defined in claim 7.

15. A method as claimed in claims 13 or 14, wherein
    (a) said electron or radical acceptor is 4-aminoantipyrine;
    (b) said peroxide is hydrogen peroxide; and
    (c) said peroxide activating agent is peroxidase.

16. A method as claimed in claim 13 or 14 or 15, further comprising reacting a precursor material with an oxidase capable of quantitatively forming peroxide in situ in the sample, whereby said precursor material may be quantitatively determined.